(19) Europäisches Patentamt
European Patent Office
Office européen des brevets

(11) Veröffentlichungsnummer : **0 352 456 B1**

# (12) EUROPÄISCHE PATENTSCHRIFT

(45) Veröffentlichungstag der Patentschrift : **18.03.92 Patentblatt 92/12**

(21) Anmeldenummer : **89110568.6**

(22) Anmeldetag : **10.06.89**

(51) Int. Cl.$^5$ : **C07C 45/74,** C07C 49/217, C07C 49/235, C07C 49/255, C07C 49/203, C07C 403/14, C07D 335/02, C07D 333/22, C07D 261/08

## (54) Verfahren zur Herstellung von alpha, beta -ungesättigten Ketonen.

Die Akte enthält technische Angaben, die nach dem Eingang der Anmeldung eingereicht wurden und die nicht in dieser Patentschrift enthalten sind.

(30) Priorität : **23.06.88 DE 3821197**

(43) Veröffentlichungstag der Anmeldung : **31.01.90 Patentblatt 90/05**

(45) Bekanntmachung des Hinweises auf die Patenterteilung : **18.03.92 Patentblatt 92/12**

(84) Benannte Vertragsstaaten : **AT BE CH DE ES FR GB IT LI NL**

(56) Entgegenhaltungen :
**WO-A-86/02065**
**DE-A- 2 107 958**
**FR-A- 2 481 701**

(73) Patentinhaber : **BASF Aktiengesellschaft**
**Carl-Bosch-Strasse 38**
**W-6700 Ludwigshafen (DE)**

(72) Erfinder : **Reissenweber, Gernot, Dr.**
**Drosselstrasse 15**
**W6737 Boehl-Iggelheim (DE)**
Erfinder : **Richarz, Winfried, Dr.**
**Koenigsberger Strasse 5**
**W-6081 Stockstadt (DE)**

EP 0 352 456 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von α,β-ungesättigten Ketonen durch Umsetzung von Aldehyden mit Acetessigsäure oder deren Salzen in Gegenwart eines primären Amins.

Aus der DE-A-31 17 271 ist ein Verfahren zur Herstellung von α,β-ungesättigten Ketonen der allgemeinen Formel

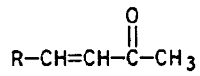

in der R einen organischen Rest bedeutet, durch Umsetzung eines entsprechenden Aldehyds R-CHO, der in α-Stellung ein Wasserstoffatom besitzen muß, mit Acetessigsäure oder einem ihrer Salze bekannt, bei dem man die Umsetzung in Anwesenheit eines sekundären, vorzugsweise nicht aliphatischen, Amins in einem zweiphasigen Reaktionsgemisch vornimmt. Allgemein ist darüber hinaus bekannt, daß gewisse Kondensationsreaktionen von Aldehyden nur in Gegenwart sekundärer Amine befriedigend verlaufen, (Organikum, Berlin 1986, Seite 466, sowie Chem. Berichte 96, 1788 f (1963)).

Der Erfindung lag daher die Aufgabe zugrunde, ein Verfahren mit einem erweiterten Anwendungsbereich zu finden und Nachteilen des aus der DE-A-31 17 271 bekannten Verfahrens abzuhelfen.

Überraschend wurde ein neues und verbessertes Verfahren zur Herstellung von α,β-ungesättigten Ketonen der allgemeinen Formel I

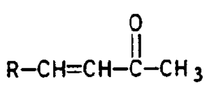

(I),

in der R einen organischen Rest bedeutet, gefunden, das in der Umsetzung eines entsprechenden Aldehyds der allgemeinen Formel R-CHO (II) mit Acetessigsäure bzw. einem ihrer Salze in einem zweiphasigen Reaktionsgemisch besteht, wobei man die Umsetzung bei einem pH-Wert von 6 bis 8 in Anwesenheit einer katalytisch wirksamen Menge eines primären Amins der allgemeinen Formel $R^1$-$NH_2$ (III), wobei $R^1$ einen organischen Rest bedeutet, vornimmt.

Das erfindungsgemäße Verfahren folgt der Reaktionsgleichung:

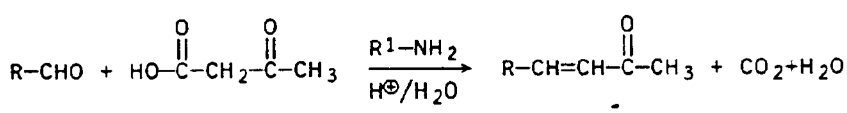


Man erkennt, daß bei deisem Verfahren das primäre Amin zwar an der Umsetzung teilnimmt, jedoch nicht verbraucht wird. Es genügt daher eine katalytisch wirksame Menge von i.a. weniger als 0,2 Mol pro Mol Aldehyd, z.B. 0,01 bis 0,2 Mol pro Mol Aldehyd.

Das Verfahren kann wie folgt durchgeführt werden:

Zur wäßrigen Lösung der Acetessigsäure bzw. einem ihrer Salze werden der Aldehyde im allgemeinen in einem Lösungsmittel und eine katalytische Menge des primären Amins gegeben und eventuell durch Zugabe einer Säure ein pH-Wert im Bereich von 6 bis 8, vorzugsweise 6,2 bis 7,2 eingestellt. Die Lösung wird bei 20 bis 100°C, vorzugsweise 30 bis 80°C, so lange gerührt (wobei durch laufende Zugabe von Säure der vorgewählte pH-Bereich eingehalten wird), bis die Umsetzung vollständig ist. Im allgemeinen genügen bei einer ausreichend hohen Temperatur 2 bis 7 Stunden. Nach Beendigung der Reaktion, die sich - bei weiterer Zugabe von Säure - am abfallenden pH-Wert erkennen läßt, wird dieser auf 2 eingestellt, und es wird dann wie üblich aufgearbeitet. Es kann zweckmäßig sein, die Reihenfolge der Zugabe der Reaktionsteilnehmer zu ändern, z.B. die wäßrige Lösung der Acetessigsäure oder eines ihrer Salze in die vorgelegte Lösung des Aldehyds bzw. Amins zu geben.

Als Salze der Acetessigsäure eignen sich Alkali- oder Erdalkalisalze, wie das Lithium-, Natrium-, Kalium- oder Calciumsalz. Als Ausgangsstoff kann z.B. handelsüblicher Acetessigsäuremethylester dienen.

Acetessigsäure bzw. deren Salz wird dabei durch Verseifung von äquimolaren Mengen Acetessigester mit einer Wäßrigen Base, wie NaOH, KOH, $Na_2CO_3$ erhalten; freiwerdender Alkohol stört in der Regel den weiteren Reaktionsverlauf nicht, und somit kann das Gemisch direkt für das erfindungsgemäße Verfahren eingesetzt werden. Bei der Herstellung von α,β-ungesättigten Ketonen, die unter sauren Bedingungen leicht Alkohol

addieren, empfiehlt es sich, den entstandenen Alkohol nach der Verseifung beispielsweise durch Destillation zu entfernen und die dann alkoholfreie wäßrige Lösung des Acetoacetats zu verwenden.

Als Aldehyde II können praktisch alle bekannten oder nach allgemeinen Standardreaktionen (Houben-Weyl, Methoden der Organischen Chemie, Bd. 7/2 (1954) und Bd. E3 (1983)) herstellbaren Aldehyde verwendet werden.

Das Molverhältnis von Acetoacetat zu Aldehyd beträgt zweckmäßig 1:1 bis 1,5:1, vorzugsweise von 1,1:1 bis 1,3:1.

Die Verwendung von 100 bis 500 ml eines mit Wasser zweckmäßig nicht mischbaren Lösungsmittels pro Mol Aldehyd empfiehlt sich in aller Regel und ist geboten, wenn der Aldehyd ein Feststoff ist.

Geeignet sind z.B. chlorierte Kohlenwasserstoffe, wie Methylenchlorid, Chloroform, Tetrachlorkohlenstoff und Dichlorethan; aromatische Kohlenwasserstoffe, wie Benzol, Toluol und Xylol; aromatische Chlorkohlenwasserstoffe, wie Chlorbenzol, oder Ether, wie Diethylether, Methyl-tert.-butylether und Diisopropylether.

Als Säure zur Regulierung des pH-Wertes eignen sich vor allem Mineralsäuren, besonders Schwefelsäure und Salzsäure.

Wenn neben dem gewünschten α,β-ungesättigten Keton I größere Mengen des entsprechenden β-Hydroxyketons IV

$$R-\overset{\overset{\displaystyle OH}{|}}{C}H-CH_2-\overset{\overset{\displaystyle O}{\|}}{C}-CH_3 \qquad (IV)$$

gebildet werden, kann die organische Phase vor dem Destillieren mit 5 Mol% Schwefelsäure, bezogen auf Aldehyd, versetzt, 2 Stunden bei 40 bis 45°C gerührt und dann wie üblich aufgearbeitet werden.

Unter den primären Aminen III sind solche bevorzugt, in denen der Rest $R^1$

– unverzweigtes oder verzweigtes $C_4$-$C_{20}$-Alkyl, besonders $C_4$-$C_{12}$-n-Alkyl, wie n-Butyl, n-Pentyl, n-Hexyl, n-Heptyl, n-Octyl, n-Decyl und n-Dodecyl,
– unverzweigtes oder verzweigtes $C_4$-$C_{20}$- Alkenyl, besonders $C_4$-$C_{12}$-n-Alkenyl,
– $C_4$-$C_{12}$-Cycloalkyl, besonders $C_4$-$C_8$-Cycloalkyl, wie Cyclobutyl, Cyclopentyl, Cyclohexyl und Cyclooctyl,
– $C_4$-$C_{20}$-Alkyl-cycloalkyl, besonders $C_4$-$C_{12}$-Alkyl-cycloalkyl, wie 2-Methylcyclohexyl,
– $C_4$-$C_{20}$-Cycloalkyl-alkyl, besonders $C_4$-$C_{12}$-Cycloalkylalkyl, wie Cyclopentyl-methyl, Cyclopentyl-ethyl, Cyclopentyl-n-butyl, Cyclopentyl-n-hexyl, Cyclohexyl-methyl, Cyclohexylethyl, Cyclohexyl-n-butyl und Cyclohexyl-n-hexyl, bedeutet. Insbesondere eignen sich primäre Amine III, in denen der Rest $R^1$ $C_4$-$C_{20}$-Alkyl oder $C_4$-$C_{20}$-Alkenyl bedeutet. Dies ist besonders bemerkenswert, weil nach der DE-A-31 17 271 gerade aliphatische und cycloaliphatische Amine weniger geeignet sein sollen.

Unter den Verfahrensprodukten I sind diejenigen bevorzugt, bei denen der Rest R folgende Bedeutung hat:

– $C_1$-$C_{20}$-Alkyl, besonders $C_1$-$C_8$-Alkyl, wie Methyl, Ethyl, n-Propyl, Isopropyl, n-Butyl, iso-Butyl, sec-Butyl, tert.-Butyl und 2,6-Dimethylheptyl,
– $C_2$-$C_{20}$-Alkenyl, besonders $C_2$-$C_8$-Alkenyl,
– $C_2$-$C_{20}$-Alkinyl, besonders $C_2$-$C_8$-Alkinyl,
– $C_2$-$C_{20}$-Alkoxy-alkyl, besonders $C_2$-$C_{12}$-Alkoxy-alkyl, wie Methoxy-methyl
– $C_3$-$C_{20}$-Alkoxy-alkenyl, besonders $C_3$-$C_{12}$-Alkoxy-alkenyl,
– $C_3$-$C_{20}$-Alkenyloxy-alkyl, besonders $C_3$-$C_{12}$-Alkenyloxy-alkyl,
– $C_4$-$C_{20}$-Alkenyloxy-alkenyl, besonders $C_4$-$C_{12}$-Alkenyloxy-alkenyl,
– $C_2$-$C_{20}$-Alkylthio-alkyl, besonders $C_2$-$C_{12}$-Alkylthio-alkyl, wie Ethylthio-2-prop-1-yl,
– $C_3$-$C_{20}$-Alkylthio-alkenyl, besonders $C_3$-$C_{12}$-Alkylthio-alkenyl,
– $C_3$-$C_{20}$-Alkenylthio-alkyl, besonders $C_3$-$C_{12}$-Alkenylthio-alkyl,
– $C_4$-$C_{20}$-Alkenylthio-alkenyl, besonders $C_4$-$C_{12}$-Alkenylthioalkenyl,
– $C_3$-$C_{20}$-Cycloalkyl, besonders $C_3$-$C_{12}$-Cycloalkyl, wie Cyclopropyl, Cyclobutyl, Cyclopentyl, Cyclohexyl, Cycloheptyl, Cyclooctyl, Cyclodecyl und Cyclododecyl,
– $C_4$-$C_{20}$-Alkyl-cycloalkyl, wie Pinan-3-yl,
– $C_3$-$C_{20}$-Cycloalkenyl, besonders $C_3$-$C_{12}$-Cycloalkenyl, wie Cyclododeca-4,8-dien-1-yl,
– $C_4$-$C_{20}$-Alkyl-cycloalkenyl, wie 2-Methylcyclohex-4-en-1-yl und 3,6,6-Trimethylcyclohexa-1,3-dien-1-yl,
– $C_4$-$C_{20}$-Bicycloalkyl, besonders $C_4$-$C_{12}$-Bicycloalkyl,
– $C_5$-$C_{20}$-Bicycloalkenyl, besonders $C_5$-$C_{12}$-Bicycloalkenyl,
– Aryl, wie Phenyl, Naphthyl und Anthranyl,
– Hetaryl, wie 2-Pyrrolyl, 3-Pyrrolyl, 2-Pyridyl, 3-Pyridyl, 4-Pyridyl,
– durch $C_1$-$C_8$-Alkyl, ein-, zwei- oder dreifach substituiertes Aryl, besonders durch $C_1$-$C_4$-Alkyl, ein-, zwei- oder dreifach substituiertes Aryl, wie p-Tolyl und 2,4,6-Trimethylphenyl,

– durch $C_1$-$C_8$-Alkoxy ein-, zwei- oder dreifach substituiertes Aryl, besonders durch $C_1$-$C_4$-Alkoxy ein-, zwei- oder dreifach substituiertes Aryl, wie p-Methoxyphenyl,
– durch Halogen ein-, zwei- oder dreifach substituiertes Aryl, besonders durch Fluor oder Chlor ein-, zwei- oder dreifach substituiertes Aryl, wie Chlorphenyl,
– durch Cyano oder Nitro ein-, zwei- oder dreifach substituiertes Aryl,
– durch $C_1$-$C_8$-Alkyl ein-, zwei- oder dreifach substituiertes Hetaryl, besonders durch $C_1$-$C_4$-Alkyl ein-, zwei- oder dreifach substituiertes Hetaryl,
– durch $C_1$-$C_8$-Alkoxy ein-, zwei- oder dreifach substituiertes Hetaryl, besonders durch $C_1$-$C_4$-Alkoxy ein-, zwei- oder dreifach substituiertes Hetaryl,
– durch Halogen ein-, zwei- oder dreifach substituiertes Hetaryl, besonders durch Fluor oder Chlor ein-, zwei- oder dreifach substituiertes Hetaryl,
– durch Cyano ein-, zwei- oder dreifach substituiertes Hetaryl,
– durch Nitro ein-, zwei- oder dreifach substituiertes Hetaryl,
– einen gesättigten heterocyclischen Rest mit 3 bis 20 Kohlenstoffatomen, besonders ein heterocyclischer Rest mit 3 bis 12 Kohlenstoffatomen, wie Thiophen-3-yl, Tetrahydropyran-3-yl und Tetrahydro-thiopyran-3-yl,
– einen gesättigten heterocyclischen Rest mit 3 bis 20 Kohlenstoffatomen und einem zusätzlichen Heteroatom aus der Gruppe Sauerstoff, Schwefel und Stickstoff,
– einen gesättigten heterocyclischen Rest mit 3 bis 20 Kohlenstoffatomen und zwei zusätzlichen Heteroatomen aus der Gruppe Sauerstoff, Schwefel und Stickstoff,
– einen ungesättigten heterocyclischen Rest mit 3 bis 20 Kohlenstoffatomen, besonders ein heterocyclischer Rest mit 3 bis 12 Kohlenstoffatomen,
– einen ungesättigten heterocyclischen Rest mit 3 bis 20 Kohlenstoffatomen und einem zusätzlichen Heteroatom aus der Gruppe Sauerstoff, Schwefel und Stickstoff, wie 5,6-Dihydro-4H-pyran-3-yl und 3-Isopropyl-isoxazol-5-yl,
– einen ungesättigten heterocyclischen Rest mit 3 bis 20 Kohlenstoffatomen und zwei zusätzlichen Heteroatomen aus der Gruppe Sauerstoff, Schwefel und Stickstoff.

Die nach dem erfindungsgemäßen Verfahren erhältlichen $\alpha,\beta$-ungesättigten Ketone I sind entweder Endprodukte (z.B. Geruchsstoffe) oder wertvolle Zwischenprodukte z.B. für herbizide Wirkstoffe (DE-A-2 822 304, DE-A- 2 439 104, DE-A-3 032 973, DE-A-3 047 924, DE-A-3 121 355, EP-A-66 195).

Beispiel 1

Herstellung des Ketons 1-(3-Tetrahydro-thiopyranyl)-1-buten-3-on

I. Herstellung nach dem erfindungsgemäßen Verfahren

a) Zu einer Mischung von 116,0 g (1 Mol) Acetessigsäuremethylester in 140 ml Wasser werden während ca. 60 min 140 g 30 %ige Natronlauge (1 Mol) getropft und anschließend ca. 5 Stunden bei 35 bis 40°C gerührt. Es entsteht eine gelbliche klare Lösung von Natriumacetoacetat.

b) Zu 396 g der so hergestellten wäßrigen Lösung, die 1 Mol Natriumacetoacetat enthält, werden 100 g (0,77 mol) Thiopyran-3-aldehyd in 100 ml Toluol und 3,8 g (0,05 mol) n-Hexylamin gegeben und der pH-Wert der wäßrigen Lösung durch Zugabe von 40 %iger Schwefelsäure auf pH 6,2 eingestellt. Das Reaktionsgemisch wird anschließend bei 50°C gerührt, wobei durch laufende Schwefelsäurezugabe der pH ständig zwischen 6,2 bis 6,4 gehalten wird. Nach Zugabe von 110 g 40 %iger Schwefelsäure innerhalb von 90 Min. wird noch 30 min nachgerührt und anschließend auf pH 2 angesäuert. Die wäßrige Phase wird abgetrennt, die organische Phase mit Wasser gewaschen und eingeengt. Man erhält 125 g eines gelblichen Öls folgender gaschromatographisch ermittelter Zusammensetzung (GC):
Tetrahydro-thiopyran-3-aldehyd 2,2 %
1-(3-Tetrahydro-thiopyranyl)-1-buten-3-on 92,3 %
1-(3-Tetrahydro-thiopyranyl)-1-hydroxy-butanon-3 3,5 %

Nach Destillation erhält man 110 g 1-(3-Tetrahydro-thiopyranyl)-1-buten-3-on (Verbindung 1; Kp. 79 bis 81°C bei 0,2 mbar) in einer Reinheit von 98 %. Die Ausbeute, bezogen auf den Aldehyd, beträgt 84 %.

II. Herstellung nach dem Verfahren gemäß DE-A-31 17 271 (zum Vergleich)

a) Unter Reaktionsführung wie in Beispiel 1, Ib unter Verwendung von Di-n-butylamin wird im pH-Bereich

zwischen 6,0 bis 6,5 umgesetzt. Die organische Phase ergibt nach 8 Stunden Reaktionszeit folgende Zusammensetzung nach GC-Analyse:
Tetrahydro-thiopyran-3-aldehyd 27,2 %
1-(3-Tetrahydro-thiopyranyl)-1-buten-3-on 46,4 %
1-(3-Tetrahydro-thio pyranyl)-1-hydroxy-butanon-3 20,5%

b) Mit Morpholin als Katalysator bei pH 6,0 bis 6,5 wird nach GC-Analyse erhalten:

| | |
|---|---|
| Tetrahydro-thiopyran-3-aldehyd | 33,1 % |
| Tetrahydro-thiopyran-3-aldehyddimethylacetal | 9,7 % |
| 1-(3-Tetrahydro-thiopyranyl)-1-buten-3-on | 32,4 % |
| 1-(3-Tetrahydro-thiopyranyl)-1-hydroxy-butanon-3 | 8,7 % |

c) Mit Pyrrolidin als Katalysator bei pH 6,5 wird nach GC-Analyse erhalten:
Tetrahydro-thiopyran-3-aldehyd 48,4 %
Tetrahydro-thiopyran-3-aldehyddimethylacetal 9,5 %
1-(3-Tetrahydro-thiopyranyl)-1-buten-3-on 29,9 %
1-(3-Tetrahydro-thiopyranyl)-1-hydroxy-butanon-3 2,3 %

Die in der folgenden Tabelle aufgeführten Verbindungen Nr. 2 bis 11 wurden entsprechend Beispiel 1, I hergestellt.

Tabelle

| Verbindung Nr. | R | prim. Amin als Katalysator | Molverhältnis Amin/Aldehyd | Ausbeute [%] | Physikalische Daten |
|---|---|---|---|---|---|
| 2 | Phenyl | n-Hexylamin | 0,03 | 91 | Fp.: 35-39°C |
| 3 | Phenyl | n-Dodecylamin | 0,05 | 88 | * |
| 4 | p-Chlorphenyl | n-Hexylamin | 0,05 | 95 | Fp.: 50-57°C |
| 5 | p-Methoxyphenyl | n-Pentylamin | 0,075 | 89 | Fp.: 70-71°C |
| 6 | p-Tolyl | n-Heptylamin | 0,05 | 95 | * |
| 7 | Thiophen-3-yl | n-Hexylamin | 0,05 | 98 | Fp.: 61-64°C |
| 8 | 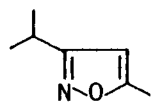 | n-Hexylamin | 0,05 | 90 | 1H-NMR ($CDCl_3$): 1,35 d (6H), 2,4 s (3H) 3,1 m (1H), 6,5 s (1H) 6,8 d (1H), 7,35 d (1H) |
| 9 | Cyclohexyl | n-Heptylamin | 0,045 | 86 | Kp.: 50-55°C/0,3 mbar |
| 10 | 4-Methylcyclohexyl | n-Hexylamin | 0,05 | 88 | 1H-NMR ($CDCl_3$): 1,95 d (3H), 1,0-2,2m (10H), 2,3 s (3H), 6,05 d (1H), 6,8 dd (1H) |
| 11 | sec.-Butyl | n-Hexylamin | 0,05 | 75 | 1H-NMR ($CDCl_3$): 1,9 d (6H), 2,8 m (1H), 2,1 t (2H), 2,25 s (3H), 6,1 d (1H) 6,8 m (1H) |

* Nachweis durch GC

Beispiel 2

1-(3-Tetrahydro-thiopyranyl)-1-buten-3-on

Bei analoger Reaktionsführung wie in Beispiel 1, I, jedoch bei einem pH-Wert von 7,0 bis 7,2, erhält man nach GC-Analyse der organischen Phase folgende Zusammensetzung:

Tetrahydro-thiopyran-3-aldehyd 2,8%
1-(3-Tetrahydro-thiopyranyl)-1-buten-3-on 80,7 %
1-(3-Tetrahydro-thiopyranyl)-1-hydroxy-butanon-3 11,3 %

Die Toluolphase wird mit 3,5 g konz. Schwefelsäure versetzt und 2 Std. bei 40 bis 45°C gerührt. Anschließend wird mit Wasser extrahiert und Toluol abgedampft. Man erhält 128 g eines Rohproduktes mit folgender Zusammensetzung (GC):

Tetrahydro-thiopyran-3-aldehyd 2,6 %
1-(3-Tetrahydro-thiopyranyl)-1-buten-3-on 89,3 %
1-(3-Tetrahydro-thiopyranyl)-1-hydroxy-butanon-3 1,2 %

**Patentansprüche**

1. Verfahren zur Herstellung von α,β-ungesättigten Ketonen der allgemeinen Formel I

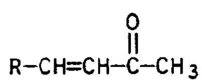 (I),

in der R einen organischen Rest bedeutet, durch Umsetzung von Aldehyden der allgemeinen Formel II

R-CHO (II)

mit Acetessigsäure oder einem ihrer Salze in Gegenwart eines Amins in einem zweiphasigen Reaktionsgemisch, dadurch gekennzeichnet, daß man die Umsetzung bei einem pH-Wert von 6 bis 8 in Anwesenheit einer katalytisch wirksamen Menge eines primären Amins der allgemeinen Formel III

$R^1$-$NH_2$ (III),

in der $R^1$ einen organischen Rest bedeutet, vornimmt.

2. Verfahren nach Anspruch 1, dadurch gekennzeichnet, daß man ein primäres Amin der Formel III verwendet, in der $R^1$ $C_4$-$C_{20}$-Alkyl oder $C_4$-$C_{20}$-Alkenyl bedeutet.

3. Verfahren zur Herstellung der α,β-ungesättigten Ketone I nach Anspruch 1, dadurch gekennzeichnet, daß der Substituent

R $C_1$-$C_{20}$-Alkyl, $C_2$-$C_{20}$-Alkenyl, $C_2$-$C_{20}$-Alkinyl, $C_2$-$C_{20}$-Alkoxyalkyl, $C_3$-$C_{20}$-Alkoxy-alkenyl, $C_3$-$C_{20}$-Alkenyloxy-alkyl, $C_4$-$C_{20}$-Alkenyloxy-alkenyl, $C_2$-$C_{20}$-Alkylthio-alkyl, $C_3$-$C_{20}$-Alkylthio-alkenyl, $C_3$-$C_{20}$-Alkenylthio-alkyl, $C_4$-$C_{20}$-Alkenylthio-alkenyl, $C_3$-$C_{20}$-Cycloalkyl, $C_3$-$C_{20}$-Cycloalkenyl, $C_4$-$C_{20}$-Bicycloalkyl, $C_5$-$C_{20}$-Bicycloalkenyl, Aryl, Hetaryl, durch $C_1$-$C_8$-Alkyl, $C_1$-$C_8$-Alkoxy, Halogen, Cyano oder Nitro ein- bis dreifach substituiertes Aryl oder Hetaryl, einen gesättigten oder ungesättigten heterocyclischen Rest mit 3 bis 20 Kohlenstoffatomen, der ein bis drei Heteroatome aus der Gruppe Sauerstoff, Schwefel und Stickstoff enthält,

bedeutet.

**Revendications**

1. Procédé de préparation de cétones α , β -insaturées, de formule générale I

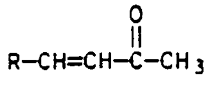 (I),

dans laquelle R représente un reste organique, par réaction d'aldéhydes de formule générale II

R-CHO (II),

avec de l'acétate d'éthyle ou l'un de ses sels, en présence d'une amine, dans un mélange réactionnel à deux phases, caractérisé par le fait que l'on effectue la réaction à un pH compris entre 6 et 8, en présence d'une quantité catalytiquement efficace d'une amine primaire de formule générale III

$R^1-NH_2$ (III),

dans laquelle $R^1$ représente un reste organique.

2. Procédé selon la revendication 1, caractérisé par le fait que l'on utilise une amine primaire de formule III dans laquelle $R^1$ représente alkyle en C 4-C 20 ou alcényle en C 4-C 20.

3. Procédé de préparation des cétones α , β -insaturées I selon la revendication 1, caractérisé par le fait que le substituant R représente alkyle en C 1-C 20, alcényle en C 2-C 20, alcynyle en C 2-C 20, alcoxyalkyle en C 2-C 20, alcoxy-C 3-C 20-alcényle, alcényloxy-C 3-C 20-alkyle, alcényloxy-C 4-C 20-alcényle, alkyl=thio-C 2-C 20-alkyle, alkylthio-C 3-C 20-alcényle, alcénylthio-C 3-C 20-alkyle, alcénylthio-C 4-C 20-alcényle, cycloalkyl en C 3-C 20, cycloalcényle en C 3-C 20, bicycloalkyle en C 4-C 20, bicycloalcényle en C 5-C 20, aryle, hétaryle, aryle ou hétaryle substitués une à trois fois par alkyle en C 1-C 8, alcoxy en C 1-C 8, halogène, cyano ou nitro, un reste hétérocyclique saturé ou insaturé à 3 à 20 atomes de carbone, qui contient un à trois hétéroatomes choisis dans le groupe oxygène, soufre et azote.

## Claims

1. A process for the preparation of an α,β-unsaturated ketone of the formula I

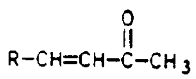

(I)

where R is an organic radical, by reacting an aldehyde of the formula II

R-CHO (II)

with acetoacetic acid or one of its salts in the presence of an amine in a two-phase reaction mixture, wherein the reaction is carried out at a pH of from 6 to 8 in the presence of a catalytic amount of a primary amine of the formula

$R^1-NH_2$ (III)

where $R^1$ is an organic radical.

2. A process as claimed in claim 1, wherein a primary amine of the formula III, where $R^1$ is $C_4$-$C_{20}$-alkyl or $C_4$-$C_{20}$-alkenyl, is used.

3. A process for the preparation of an α,β-unsaturated ketone I as claimed in claim 1, wherein R is $C_1$-$C_{20}$-alkyl, $C_2$-$C_{20}$-alkenyl, $C_2$-$C_{20}$-alkynyl, $C_2$-$C_{20}$-alkoxyalkyl, $C_3$-$C_{20}$-alkoxyalkenyl, $C_3$-$C_{20}$-alkenyloxyalkyl, $C_4$-$C_{20}$-alkenyloxyalkenyl, $C_2$-$C_{20}$-alkylthioalkyl, $C_3$-$C_{20}$-alkylthioalkenyl, $C_3$-$C_{20}$-alkenylthioalkyl, $C_4$-$C_{20}$-alkenylthioalkenyl, $C_3$-$C_{20}$-cycloalkyl, $C_3$-$C_{20}$-cycloalkenyl, $C_4$-$C_{20}$-bicycloalkyl, $C_5$-$C_{20}$-bicycloalkenyl, aryl, hetaryl, aryl or hetaryl which is monosubstituted to trisubstituted by $C_1$-$C_8$-alkyl, $C_1$-$C_8$-alkoxy, halogen, cyano or nitro, or a saturated or unsaturated heterocyclic radical of 3 to 20 carbon atoms which contains from one to three heteroatoms from the group consisting of oxygen, sulfur and nitrogen.